(19) Europäisches Patentamt
European Patent Office
Office européen des brevets

(11) Veröffentlichungsnummer: **0 306 853 B1**

(12) **EUROPÄISCHE PATENTSCHRIFT**

(45) Veröffentlichungstag der Patentschrift: **10.06.92**

(51) Int. Cl.[5]: **A61K 7/26**, A61K 7/32

(21) Anmeldenummer: **88114354.9**

(22) Anmeldetag: **02.09.88**

Die Akte enthält technische Angaben, die nach dem Eingang der Anmeldung eingereicht wurden und die nicht in dieser Patentschrift enthalten sind.

(54) **Kosmetisches Präparat mit germiziden Eigenschaften.**

(30) Priorität: **11.09.87 DE 3730522**

(43) Veröffentlichungstag der Anmeldung:
**15.03.89 Patentblatt 89/11**

(45) Bekanntmachung des Hinweises auf die Patenterteilung:
**10.06.92 Patentblatt 92/24**

(84) Benannte Vertragsstaaten:
**CH DE FR GB LI**

(56) Entgegenhaltungen:
**DE-A- 1 617 556**
**DE-A- 2 637 862**
**FR-A- 2 133 237**

**CHEMICAL ABSTRACTS, Band 40, Nr. 22, 20. November 1946, Spalte 7281, letzter Absatz - Spalte 7282, erster Absatz, Columbus, Ohio, US; G. DEL VECCHIO et al.: "Antibiotic action of quinones. I. Antibiotic activity against Staphylococcus pyogenes aureus in vitro", & BOLL. SOC. ITAL. BIOL. SPER. 21, 1-3(1946)**

(73) Patentinhaber: **Wixforth, Bruno**
**Wilhelm-Böhmert-Strasse 17**
**W-2800 Bremen(DE)**

(72) Erfinder: **Wixforth, Bruno**
**Wilhelm-Böhmert-Strasse 17**
**W-2800 Bremen(DE)**

(74) Vertreter: **Kraus, Walter, Dr. et al**
**Patentanwälte Kraus, Weisert & Partner**
**Thomas-Wimmer-Ring 15**
**W-8000 München 22(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

SEIFE-OELE-FETTE-WACHSE, Band 107, Nr. 20, Dezember 1981, Seiten 623-625, Verlag für Chemische Industrie, H. Zoilkowsky KG, Augsburg, DE; "Verwendung von Herbasol-Extrakten in der Kosmetik"

CHEMICAL ABSTRACTS, Band 76, Nr. 16, 17. April 1972, Seite 298, Nr. 89952b, Columbus, Ohio, US; M. AHMAD et al.: "Chemical extractives from walnut bark (Juglans regia) and their effects on the cleansing of tartar deposits", PAK. J. FOREST. 1971, 21(3), 325-7

CHEMICAL ABSTRACTS, Band 89, Nr. 9, 28. August 1978, Seite 103, Nr. 71511r, Columbus, Ohio, US; W.M. KRAJCI et al.: "The inhibition of various micro-organisms by crude walnut hull extracts and juglone", & MICROBIOS. LETT. 1977, 4(15), 175-81

Wagner H., Pharmazeutische Biologie, Gustav Fischer Verlag, 1985

## Beschreibung

Der in der vorliegenden Anmeldung verwendete Ausdruck "kosmetisches Präparat" umfaßt kosmetische Mittel, Kosmetika und Körperpflegemittel.

Bekanntlich ist die Zahnkaries die derzeit häufigste Zivilisationskrankheit, von der in den Industriestaaten über 90% der Bevölkerung befallen sind. Trotz aller Anstrengungen, mit wirksamen Mitteln in die Genese dieser Krankheit einzugreifen, ist ein dauerhafter und eklatanter Erfolg zur Zeit nicht zu verzeichnen. Am wirksamsten haben sich bisher Fluoridzusätze zu Zahnpflegemitteln oder zum Trinkwasser erwiesen, weil diese die Zahnoberfläche härten und gegen die von der Bakterienpopulation der Plaque gebildeten organischen Säuren widerstandsfähiger machen. Der direkte und wirksamste Eingriff in die Kariesgenese, nämlich die Abtötung der säurebildenden Plaque-Mischpopulation , ist nach dem derzeitigen Stande der Technik sicher nur mit Antibiotika möglich, deren laufender Gebrauch, wie er hierfür notwendig wäre, bekanntlich aus Gründen der Resistenzbildung äußerst problematisch ist.

In Nordafrika wurde beobachtet, daß ein erheblicher ärmerer Teil bestimmter Bevölkerungsgruppen, vor allem im Atlas-Gebirge, zur Zahnpflege getrocknete Pflanzenteile von JUGLANS REGIA kaute. Diese Menschen zeichnen sich durch einen bemerkenswert guten Zahnerhaltungszustand aus.

Die Verwendung der getrockneten JUGLANS REGIA, wie sie in Nordafrika verwendet wird, ist in den europäischen Industrienationen aus geschmacklichen und ästhetischen Gründen in dieser Form nicht möglich.

Bei deodorierenden kosmetischen Erzeugnissen werden nach dem Stand der Technik chemisch komplizierte, synthetische, keimtötende Stoffe, zum Beispiel bei einem Deodorant-Gel in Mengen von ca. 5.000 mg/kg, eingesetzt. Bei diesen synthetischen Stoffen ist jedoch zu bemerken, daß ein meßbarer Anteil der Bevölkerung auf diese allergisch reagieren kann. Außerdem lehnt ein großer Teil der Bevölkerung, der noch im Zunehmen begriffen ist, aus welchen Gründen auch immer, sogenannte "künstliche" Stoffe in Konsumprodukten ab und bevorzugt "natürliche" Stoffe mit entsprechender Wirkung.

Wagner H., Pharmazeutische Biologie, Drogen und ihre Inhaltsstoffe, Gustav Fischer Verlag, 1985, beschreibt die pharmazeutische Verwendung von Walnußblättern (Juglandis folium). Der als Droge formulierte Pflanzenbestandteil sind die getrockneten Fiederblättchen des Walnußbaumes. Daneben wird auf die kosmetische Verwendung von Extrakten aus den Schalen der unreifen Walnußfrüchte als bräunender Zusatz zu Sonnenölen hingewiesen. Die Verwendung unterirdischer Walnußwurzelrinde, wie es gemäß vorliegender Erfindung der Fall ist, ist weder beschrieben noch nahegelegt.

Gegenstand der DE-OS 26 37 862 ist ein pulverförmiges Zahnpflegemittel, das aus fein pulverisierter Kohle besteht. Daneben kann dieses Zahnpflegemittel noch fein pulverisierte Wirkstoffe der Walnußrinde enthalten. Dabei enthält diese Druckschrift keinen Hinweis darauf, um welche Art der Walnußrinde es sich hier handelt. Auch ist das Problem der Stabilität des Wirkstoffs in keiner Weise erwähnt.

In Seife-Öle-Fette-Wachse, Band 107 (1981) ist die Verwendung von Walnußschalen, d.h. die handelsüblichen, dunkelbraunen, getrockneten, grünen Fruchthüllen der Walnüsse, wie sie zu Typagen verwendet werden, beschrieben. Bei keiner der aufgeführten kosmetischen Formulierungen ist Walnußwurzelrinde als Wirkstoff aufgeführt.

Chemical Abstracts, Band 76, Nr. 16, 17. April 1972, Seite 298, Nr. 89952b, betrifft die Verwendung von Walnußrindenextrakt in Zahnpasta.

Der vorliegenden Erfindung liegt die Aufgabe zugrunde, ein kosmetisches Präparat mit germiziden Eigenschaften zur Verfügung zu stellen, welches ein verträgliches Germizid in geringer Konzentration und nicht einen chemisch komplizierten, synthetischen Stoff als Germizid enthält. Das kosmetische Präparat soll leicht verträglich sein und als Germizid einen Naturstoff enthalten.

Erfindungsgemäß soll insbesondere eine lagerstabile, den Nord- und Mitteleuropäern gewohnte Zubereitungsform für antikariogene Zahnpflegemittel zur Verfügung gestellt werden. Erfindungsgemäß sollen deodorierende kosmetische Erzeugnisse zur Verfügung gestellt werden, welche germizide Eigenschaften besitzen und kein chemisch kompliziertes, synthetisches Germizid enthalten.

Erfindungsgemäß sollen kosmetische Präparate zur Verfügung gestellt werden, insbesondere Zahn- und Mundpflegemittel, welche Pflanzenteile von JUGLANDACEAE-Arten als Germizid enthalten.

Gegenstand der Erfindung ist ein kosmetisches Präparat mit germiziden Eigenschaften, das dadurch gekennzeichnet ist, daß es 5-Hydroxy-1,4-naphthochinon in Form von Pflanzenteilen, die einen Gehalt an 5-Hydroxy-1,4-naphthochinon aufweisen und deren Wassergehalt auf höchstens 0,3 bis 0,5 % abgesenkt wurde, enthält, wobei die Pflanzenteile Wurzelrinden von Juglandaceae-Arten sind.

Gegenstand der Erfindung ist weiterhin ein kosmetisches Präparat mit germiziden Eigenschaften, das dadurch gekennzeichnet ist, daß es 5-Hydroxy-1,4-naphthochinon in Form eines Extraktes enthält, wobei der Extrakt durch Extraktion von Wurzelrinden von Juglandaceae-Arten, die einen Gehalt an 5-Hydroxy-1,4-naphthochinon aufweisen, gewonnen worden ist.

Die Erfindung betrifft weiterhin die Verwendung von 5-Hydroxy-1,4-naphthochinonals Germizid für kosmetische Präparate in Form von Pflanzenteilen, die einen Gehalt an 5-Hydroxy-1,4-naphthochinon aufweisen und deren Wassergehalt auf höchstens 0,3 bis 0,5 % abgesenkt wurde, und wobei die Pflanzenteile Wurzelrinden von Juglandaceae-Arten sind, sowie die Verwendung von 5-Hydroxy-1,4-naphthochinon in Form eines Extraktes, wobei der Extrakt durch Extraktion von Wurzelrinden von Juglandaceae-Arten, die einen Gehalt an 5-Hydroxy-1,4-naphthochinon aufweisen, gewonnen worden ist.

Die nähere Untersuchung zeigte, daß der Hauptwirkstoff von JUGLANDACEAE-Arten, das 5-Hydroxy-1,4-naphthochinon, das auch als JUGLON bezeichnet wird, der folgenden Formel I ist:

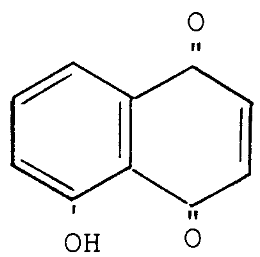

(I)

Die Untersuchungen haben gezeigt, daß das 5-Hydroxy-1,4-naphthochinon gegen bestimmte äußere Einflüsse instabil ist und zur Bildung von Kondensationsprodukten, wie bei Chinonen allgemein üblich, neigt.

Überraschenderweise wurde gefunden, daß die Kondensationsreaktion des Naphthochinonderivats, die in vitro mit einer zunehmenden Abnahme der germiziden Wirkung einhergeht, unterdrückt bzw. verzögert werden kann, wenn diese Verbindung kosmetischen Produkten in geringer Menge beigemischt wird. Es zeigte sich insbesondere, daß die Kondensationsreaktion in der getrockneten JUGLANDACEAE-Droge unterdrückt bzw. verzögert werden kann, wenn der Feuchtigkeitsgehalt der Trockenpflanze bzw. der Pflanzenteile auf höchstens 0,3 bis 0,5% abgesenkt wurde. Die gesundheitliche Unbedenklichkeit der Verbindung der Formel I als auch die Verwendung von JUGLANDACEAE-Pflanzen oder -Pflanzenteilen dürfte durch den wahrscheinlich jahrhundertlangen Gebrauch in Nordafrika als erwiesen gelten. In der Tat ist 5-Hydroxy-1,4-naphthochinon, d.h. der Wirkstoff aus JUGLANS REGIA, nicht unphysiologisch. Viele Stoffe aus der Gruppe der substituierten Naphthochinone haben eine Vitamin-K-Wirksamkeit. Vitamin $K_1$ ist ebenfalls ein substituiertes Naphthochinon. Zudem werden Extrakte aus JUGLANS REGIA seit langer Zeit als sogenanntes Typagen zur Färbung von Spirituosen eingesetzt.

Erfindungsgemäß wird das 5-Hydroxy-1,4-naphthochinon in Form von Wurzelrinden von JUGLANDACEAE-Arten oder eines Extraktes davon verwendet. Als JUGLANDACEAE-Arten können die Arten, die in Heinz A. Hoppe "Taschenbuch der Drogenkunde", Walter de Gruyter, Berlin, New York 1981, S. 155 bis 156, aufgeführt sind, verwendet werden. Erfindungsgemäß ist es besonders bevorzugt, JUGLANS REGIA L. und JUGLANS NIGRA L. zu verwenden. Erfindungsgemäß können die Wurzelrinden in zerkleinerter, vorzugsweise vermahlener, Form oder Pflanzenteile verwendet werden.

Es ist erfindungsgemäß bevorzugt, die Wurzelrinde in zerkleinerter Form oder gemahlen zu verwenden.

Wie oben ausgeführt wurde, wird der Wassergehalt der Trockenpflanze auf höchstens 0,3 bis 0,5% abgesenkt. Erfindungsgemäß ist es besonders bevorzugt, die gemahlene Trockendroge vor ihrer Verwendung mit einem hydrophobierenden Stoff zu behandeln, vorzugsweise zu besprühen. Erfindungsgemäß werden die gemahlenen, getrockneten Wurzelrinden von JUGLAN-DACEAE-Arten, vorzugsweise JUGLANS REGIA, in an sich bekannter Weise mit 0,5 bis 2,0%, vorzugsweise 0,5 bis 1,0%, eines Hydrophobierungsmittels besprüht. Als Hydrophobierungsmittel eignen sich die an sich bekannten wasserabstoßenden Flüssigkeiten. Es ist besonders bevorzugt, Siliconöl zu verwenden. Beispiele sind fette Öle (Glycerinester der Fettsäuren), Paraffinöle und andere wasserabstoßende Stoffe. Die Hydrophobierung der JUGLANS-Wurzelrinden mit dem hydrophobierenden Stoff kann beispielsweise durch Tränkung der Wurzelrinden oder durch Besprühen durchgeführt werden. Erfindungsgemäß können die Wurzelrinden der JUGLANDACEAE-Arten auch extrahiert werden, und der erhaltene Extrakt kann der Grundrezeptur des kosmetischen Präparats zugesetzt werden. Erfindungsgemäß werden die oben erwähnte getrocknete Wurzelrinde von JUGLANDACEAE-Arten in zerkleinerter Form, vorzugsweise als Pulver, mit einem Extraktionsmittel aus 70 bis 100% Ethanol und 0 bis 30% Wasser extrahiert.

Der erfindungsgemäße alkoholische Extrakt aus der Trockendroge wird durch Vermischen von zum Beispiel 100 g getrockneter, gemahlener JUGLANS-REGIA-Wurzelrinde und 1000 ml Ethanol, das 0 bis 30

Vol.-%, vorzugsweise 10 bis 25 Vol.-%, besonders bevorzugt 15 Vol.-%, Wasser enthält, hergestellt, indem man diese Mischung in Dunkelheit bei Raumtemperatur 12 bis 24 Stunden unter gelegentlichem Umrühren stehenläßt. Anschließend wird filtriert, und der Extrakt wird dunkel und kühl aufgehoben.

Ein Extrakt, der mit reinem Wasser oder reinem Ethanol als Extraktionsmittel hergestellt wurde, ergab überraschenderweise nach Zugabe zu bestimmten kosmetischen Zubereitungen im Agar-Diffusionstest unbefriedigende Ergebnisse, d.h. er ergab keine oder nur kleine Hemmhöfe. überraschenderweise zeigte sich, daß ein Extraktionsmittel aus 70 bis 90% Ethanol und 10 bis 30% Wasser einen Extrakt ergab, der, wenn er in kosmetischen Zubereitungen verwendet wurde, wirksam war. Diese kosmetischen Zubereitungen zeigten im Agar-Diffusionstest überraschend große Hemmhöfe bei Plaque-Mischkulturen und Mischkulturen aus der Achselhöhle auf "Total-Count"-Nährböden.

Der erfindungsgemäß hergestellte Extrakt ist bei dunkler und kühler Lagerung auch nach mehreren Monaten unvermindert wirksam.

Überraschenderweise zeigte sich, daß nur sehr geringe Mengen an 5-Hydroxy-1,4-naphthochinon benötigt werden. Wird reines 5-Hydroxy-1,4-naphthochinon eingesetzt, so wird es vorzugsweise als alkoholische oder alkoholisch-wäßrige Lösung dem kosmetischen Präparat zugegeben. Es wird so verwendet, daß das kosmetische Präparat 0,001 bis 0,5 Gew.-%, vorzugsweise 0,005 bis 0,1 Gew.-%, an 5-Hydroxy-1,4-naphthochinon, bezogen auf den Wassergehalt des kosmetischen Präparats, enthält. Bei der Herstellung der erfindungsgemäßen kosmetischen Präparate werden auch nur sehr geringe Mengen an Trockenpflanzen oder an Extrakt benötigt, um die erwünschten Wirkungen zu erzielen. Die Wurzelrinden oder der Extrakt werden dem kosmetischen Präparat in solchen Mengen zugegeben, daß der Gehalt an 5-Hydroxy-1,4-naphthochinon 0,001 bis 0,5 Gew.-%, bezogen auf den Wassergehalt des kosmetischen Präparats, beträgt.

Zur Herstellung eines antikariogenen Kaugummis zu einer an sich bekannten Kaugummi-Rezeptur werden beispielsweise 0,5 bis 10% getrockneter und erfindungsgemäß hydrophobierter JUGLANS-REGIA-Wurzelrinden oder nichthydrophobierter Wurzelrinden zugemischt und nach bekannten Verfahren zu einem Fertigprodukt verarbeitet.

In einem antikariogenen Mundwasser, das in der Regel zur Benutzung mit sehr viel Wasser verdünnt wird, werden hier 10 bis 100 Vol.-% des reinen erfindungsgemäßen Extrakts mit etherischen Ölen aromatisiert eingesetzt, so daß der Juglongehalt im Mundwasser 0,1 bis 5,0% beträgt.

Deodorierende Erzeugnisse enthalten als Wirksubstanz 0,1 bis 10% getrocknete, hydrophobierte und gemahlene JUGLANS-REGIA-Wurzelrinden oder - je nach Zubereitungsform des Endproduktes - 1 bis 100% erfindungsgemäßen Extrakt, bezogen auf das Gesamtprodukt.

Erfindungsgemäße kosmetische Präparate sind Mittel der Hygiene, wie Seifen, Intim-, Haar-, Kopf-, Mund- und Zahnpflegemittel, Desodorantien sowie Produkte der pflegenden und dekorativen Kosmetik, Sonnenschutzmittel, Hautpflegemittel, Gesichtspackungen und dergleichen. Auf die Grundrezepturen dieser Mittel, die an sich bekannt sind, soll nicht weiter eingegangen werden. Diesen Mitteln wird erfindungsgemäß 5-Hydroxy-1,4-naphthochinon aus Wurzelrinden von JUGLANDACEAE-Arten oder ein Extrakt aus Wurzelrinden von JUGLANDACEAE-Arten in geringer Menge beigesetzt. Bevorzugte erfindungsgemäße kosmetische Präparate sind Zahnpflegemittel zur Kariesprophylaxe und Deo-Erzeugnisse, da eine relativ geringe Dosierung eine maximale germizide Wirkung ausübt. Erfindungsgemäß werden besonders Kaugummi zur Zahnpflege, Zahnpasten, ein Zahnpulver oder ein Mundwasser sowie Desodorantien und Fußpuder bevorzugt.

Durch Einsatz des durch Hydrophobierung stabilisierten, gepulverten und getrockneten Pflanzenmaterials und/oder eines Extrakts mit wasserhaltigem Ethanol lassen sich eine Vielzahl kosmetischer Präparate herstellen, wie Zahnpasten, Kaugummi zur Zahnpflege, Zahnpulver, Mundwasser-Deo-Produkte, Fußpuder und andere mehr. Diese Produkte sind besonders bevorzugt.

Wie sich in vitro im Agar-Diffusionstest erwiesen hat, sind die erfindungsgemäßen Zahnpflegeprodukte besonders wirksam gegen die Bakterienpopulation der Zahn-Plaque und die erfindungsgemäßen kosmetischen deodorierenden Erzeugnisse gegen die Mischpopulation der Körperoberfläche, die die unangenehmen Geruchsbildungen der Körperoberfläche verursacht.

Im Agar-Diffusionstest und anderen in-vitro-Versuchen konnte überraschenderweise eine überraschend hohe Wirksamkeit sowohl der getrockneten und gemahlenen Droge als auch des Extraktes aus der Droge festgestellt werden. So ergab im Agar-Diffusionstest ein Stück getrockneter JUGLANS REGIA von 100 mg Gewicht nach Beimpfung des Nährbodens mit einer Plaque-Mischkultur einen Hemmhof nach Bebrütung von 35 mm Durchmesser. Nach einer Beimpfung des Nährbodens mit einer Mischkultur aus der Achselhöhle ergab sich unter den gleichen Bedingungen ein Hemmhof mit einem Durchmesser von 38 mm. In einer Submerskultur zeigte sich, daß lediglich 1000 mg getrockneter und feingemahlener JUGLANS REGIA in 1 kg Substrat ausreichten, sämtliche Keime des vorher beimpften Substrates abzutöten. Der gleiche Effekt

wurde erzielt, wenn nur 32,5mg Juglon zugemischt Wurden.

Verglichen mit den Kosten für synthetische, in wesentlich höherer Konzentration einzusetzenden konventionellen bakteriziden Wirkstoffen sind diejenigen für die gemahlene, getrocknete JUGLANS-REGIA-Droge als sehr niedrig zu bezeichnen.

Beispiel eines Einsatzes des reinen Wirkstoffes aus JUGLANS REGIA (JUGLON):

Ein Cellulosefilzstückchen von ca 2 mm Dicke und einer Fläche von 5 x 5 mm wurde mit 0,1 ml einer 0,25%igen JUGLON-Lösung in Alkohol getränkt und an der Luft getrocknet. Dieses so präparierte Cellulosefilzstückchen wurde auf einen Total-count-Nährboden aufgebracht, der vorher über die ganze Oberfläche (Durchmesser 9 mm) mit folgenden Mischpopulationen beimpft war:

1. Zahnplaque-Population
2. Achselhöhlen-Population
3. Saccharomyces cerevisiae (pH 7)
4. Saccharomyces cerevisiae (pH 3,4)

Die sich nach einer Bebrütung von ca. 18 Stunden bei 25°C ausbildenden Hemmhöfe hatten folgende Durchmesser:

1:30 mm
2:46 mm
3:30 mm
4:56 mm.

Die folgenden Beispiele erläutern die Erfindung.

**Beispiel 1**

Rezepturbeispiel für einen Kaugummi:

In 1 kg einer an sich bekannten Kaugummimischung werden 10 bis 80 g feingemahlener getrockneter Pflanzenmaterialien von zum Beispiel JUGLANS-REGIA-Wurzelrinde in der Produktionsphase, in der das Aroma zugemischt wird, in einem Spezialkneter gründlich untergemischt. Es ist darauf zu achten, daß bald nach der Zugabe des JUGLANS-Pulvers die Masse, wie im Verfahren vorgeschrieben, gekühlt wird.

Mischbeispiel:

| | |
|---|---|
| 15 bis 25% | Kaugummi-Grundmasse ("gum-base") |
| 20 bis 30% | Glucosesirup |
| 50 bis 60% | Puderzucker |
| 0,5 bis 8% | getrocknetes JUGLANS-Pulver |
| 1 bis 2% | üblicher Weichmacher (z.B. Glycerin) |
| 3 bis 6% | Restwassergehalt. |

Anstelle des Glucosesirups und des Puderzuckers können für "zuckerfreie" Rezepturen auch die Zuckeralkohole Mannit, Xylit und Sorbit, "Palatinit" und andere sowie künstliche Süßstoffe, wie Saccharin, Cyclamat, Acesulfam-K und Aspartame, eingesetzt werden.

Diese Rezeptur stellt nur ein Beispiel dar. Die Praxis der Kaugummi-Formulierung in aller Welt hat vielfältige Abweichungen hiervon hervorgebracht, die jedoch alle in etwa der obigen Rezeptur entsprechen.

Zur Erhöhung der Haltbarkeit des JUGLANS-Wirkstoffes kann das JUGLANS-Pulver vor der Beimischung erfindungsgemäß mit einem hydrophobierenden Stoff behandelt werden.

Die oben beschriebene Beimischung an JUGLANS-Pulver entspricht in etwa einem Gehalt an 5-Hydroxy-1,4-naphthochinon von 16 bis 260 mg pro 100 g Kaugummi-Fertigmischung. In dieser Konzentration kann das 5-Hydroxy-1,4-naphthochinon auch als reiner wirkstoff anstelle des JUGLANS-Pulvers beigemischt werden, um den gleichen Effekt zu erzielen.

**Beispiel 2**

Rezepturbeispiel für ein Deodorantgel, mit einem "Deo-Roller" aufgetragen:

100 ml einer in den Grundzügen an sich bekannten Deodorant-Gel-Rezeptur setzen sich zusammen aus:

| | |
|---|---|
| 1 bis 100 ml | eines Ethanol-Wasser-Extraktes aus frischer oder getrockneter JUGLANS-REGIA-Wurzelrinde, |
| 0 bis 99 ml | Ethanol-Wasser-Mischung, |
| 0,1 bis 10% | Methylcellulose oder Polyoxyethylen-Polyoxypropylen-Blockpolymerisat, |
| q.s. | Parfümöl |

Andere technologisch oder chemisch bedingte Zusätze können hinzugefügt werden. Anstelle des Extraktes aus JUGLANS-Rinde kann auch mit dem gleichen Effekt eine ethanolisch-wäßrige Lösung von 5-Hydroxy-1,4-naphthochinon mit insgesamt 16 bis 260 mg Wirkstoff in 100 ml Deo-Gel verwendet werden. Anstelle des Ethanols können auch andere geeignete Lösungsmittel zur Anwendung kommen.

**Beispiel 3**

Rezepturbeispiel für einen Fußpuder:

100 g eines in den Grundzügen bekannten Fußpuders setzen sich zusammen aus:

| | |
|---|---|
| Talcum | 70,0 g |
| Magnesiumhydroxid-carbonat | 19,2 bis 29,1 g |
| Allantoin | 0,5 g |
| Parfümöl | 0,3 g |
| JUGLANS-Wurzelrinden-Pulver | 0,1 bis 10 g |

Anstelle des JUGLANS-Wurzelrinden-Pulvers kann auch mit dem gleichen Effekt eine Mischung aus 3,25 mg bis 400 mg 5-Hydroxy-1,4-naphthochinon mit einer geeigneten Trägersubstanz, zum Beispiel Talcum, eingesetzt werden.

**Patentansprüche**

**1.** Kosmetisches Präparat mit germiziden Eigenschaften, dadurch **gekennzeichnet,** daß es 5-Hydroxy-1,4-naphthochinon in Form von Pflanzenteilen enthält, die einen Gehalt an 5-Hydroxy-1,4-naphthochinon aufweisen und deren Wassergehalt auf höchstens 0,3 bis 0,5 % abgesenkt wurde, wobei die Pflanzenteile Wurzelrinden von JUGLANDACEAE-Arten sind.

**2.** Kosmetisches Präparat mit germiziden Eigenschaften, dadurch **gekennzeichnet,** daß es 5-Hydroxy-1,4-naphthochinon in Form eines Extraktes enthält, wobei der Extrakt durch Extraktion von Wurzelrinden von JUGLANDACEAE-Arten, die einen Gehalt an 5-Hydroxy-1,4-naphthochinon aufweisen, gewonnen worden ist.

**3.** Kosmetisches Präparat nach Anspruch 1, dadurch **gekennzeichnet,** daß die Wurzelrinden von JUGLANDACEAE-Arten von getrockneter JUGLANS REGIA L. oder JUGLANS NIGRA L., in fein gemahlener Form sind.

**4.** Kosmetisches Präparat nach Anspruch 1 oder 3, dadurch **gekennzeichnet,** daß es Wurzelrinden enthält, die mit einem hydrophobierenden Stoff behandelt wurden.

**5.** Kosmetisches Präparat nach Anspruch 1, dadurch **gekennzeichnet,** daß es 0,001 bis 0,5 Gew.-% 5-Hydroxy-1,4-naphthochinon, bezogen auf den Wassergehalt des kosmetischen Präparats, enthält.

**6.** Kosmetisches Präparat nach einem der Ansprüche 1 bis 4, dadurch **gekennzeichnet,** daß es die Wurzelrinden oder den Extrakt in solcher Menge enthält, daß der Gehalt an 5-Hydroxy-1,4-naphthochinon 0,001 bis 0,5 Gew.-%, bezogen auf den Wassergehalt des kosmetischen Präparats, beträgt.

7. Kosmetisches Präparat nach einem der Ansprüche 1 bis 6, dadurch **gekennzeichnet,** daß es in Form eines Zahn- und Mundpflegemittels, eines Desodorants oder eines Fußpuders vorliegt.

8. Kosmetisches Präparat nach Anspruch 7, dadurch **gekennzeichnet,** daß das Zahn- und Mundpflegemittel eine Zahnpasta, ein Kaugummi zur Zahnpflege, ein Zahnpulver oder ein Mundwasser ist.

9. Verfahren zur Herstellung eines kosmetischen Präparats mit germiziden Eigenschaften nach mindestens einem der Ansprüche 1 bis 8, dadurch **gekennzeichnet,** daß man der an sich bekannten Grundrezeptur des kosmetischen Präparats Wurzelrinden von JUGLANDACEAE-Arten, die einen Gehalt an 5-Hydroxy-1,4-naphthochinon aufweisen, oder einen daraus gewonnenen Extrakt beimischt.

10. Verwendung von 5-Hydroxy-1,4-naphthochinon in Form von Pflanzenteilen, die einen Gehalt an 5-Hydroxy-1,4-naphthochinon aufweisen, und deren Wassergehalt auf höchstens 0,3 bis 0,5 % abgesenkt wurde, als Germizid für kosmetische Präparate, wobei die Pflanzenteile Wurzelrinden von JUGLANDACEAE-Arten sind.

11. Verwendung von 5-Hydroxy-1,4-naphthochinon in Form eines Extraktes, wobei der Extrakt durch Extraktion von Wurzelrinden von JUGLANDACEAE-Arten, die einen Gehalt an 5-Hydroxy-1,4-naphthochinon aufweisen, gewonnen worden ist.

12. Verwendung nach Anspruch 10, dadurch **gekennzeichnet,** daß als Wurzelrinden von JUGLANDACEAE-Arten Wurzelrinden von getrockneter JUGLANS REGIA L. oder JUGLANS NIGRA L. in fein gemahlener Form verwendet werden.

13. Verwendung nach Anspruch 10 oder 12 bis 16, dadurch **gekennzeichnet,** daß Wurzelrinden verwendet werden, die mit einem hydrophobierenden Stoff behandelt wurden.

14. Verwendung nach Anspruch 10, dadurch **gekennzeichnet,** daß 0,001 bis 0,5 Gew.-% 5-Hydroxy-1,4-naphthochinon, bezogen auf den Wassergehalt des kosmetischen Präparats, verwendet werden.

15. Verwendung nach einem der Ansprüche 10 bis 13, dadurch **gekennzeichnet,** daß die Wurzelrinden oder der Extrakt in solcher Menge verwendet werden, daß der Gehalt an 5-Hydroxy-1,4-naphthochinon 0,001 bis 0,5 Gew.-%, bezogen auf den Wassergehalt des kosmetischen Präparats, beträgt.

16. Verwendung nach einem der Ansprüche 10 bis 15, dadurch **gekennzeichnet,** daß das 5-Hydroxy-1,4-naphthochinon in einem Zahn- und Mundpflegemittel, einem Desodorant oder einem Fußpuder verwendet wird.

17. Verwendung nach Anspruch 16, dadurch **gekennzeichnet,** daß das Zahn- und Mundpflegemittel eine Zahnpasta, ein Kaugummi zur Zahnpflege, ein Zahnpulver oder ein Mundwasser ist.

## Claims

1. A cosmetic preparation having germicidal properties, characterized in that it contains 5-hydroxy-1,4-naphthoquinone in the form of parts of plants which contain 5-hydroxy-1,4-naphthoquinone and of which the water content has been reduced to at most 0.3 to 0.5%, the plant parts being root bark of JUGLANDACEAE species.

2. A cosmetic preparation having germicidal properties, characterized in that it contains 5-hydroxy-1,4-naphthoquinone in the form of an extract, the extract having been obtained by extraction of root bark of JUGLANDACEAE species containing 5-hydroxy-1,4-naphthoquinone.

3. A cosmetic preparation as claimed in claim 1, characterized in that the root bark of JUGLANDACEAE species is root bark of dried JUGLANS REGIA L. or JUGLANS NIGRA L. in finely ground form.

4. A cosmetic preparation as claimed in claim 1 or 3, characterized in that it contains root bark which has been treated with a hydrophobicizing agent.

5. A cosmetic preparation as claimed in claim 1, characterized in that it contains 0.001 to 0.5% by weight 5-hydroxy-1,4-naphthoquinone, based on the water content of the cosmetic preparation.

6. A cosmetic preparation as claimed in any of claims 1 to 4, characterized in that it contains the root bark or the extract in such quantities that the content of 5-hydroxy-1,4-naphthoquinoneis from 0.001 to 0.5% by weight, based on the water content of the cosmetic preparation.

7. A cosmetic preparation as claimed in any of claims 1 to 6, characterized in that it is in the form of a dental or oral hygiene preparation, a deodorant or a foot powder.

8. A cosmetic preparation as claimed in claim 7, characterized in that the dental or oral hygiene preparation is a tooth paste, a dental chewing gum, a tooth powder or a mouthwash.

9. A process for the preparation of the cosmetic preparation having germicidal properties claimed in at least one of claims 1 to 8, characterized in that root bark of JUGLANDACEAE species containing 5-hydroxy-1,4-naphthoquinone or an extract obtained therefrom is added to the basic formulation known **per se** of the cosmetic preparation.

10. The use of 5-hydroxy-1,4-naphthoquinone in the form of parts of plants which 5-hydroxy-1,4-naphthoquinone and of which the water content has been reduced to at most 0.3 to 0.5% as a germicide for cosmetic preparations, the plant parts being root bark of JUGLANDACEAE species.

11. The use of 5-hydroxy-1,4-naphthoquinone in the form of an extract, the extract having been obtained by extraction of root bark of JUGLANDACEAE species containing 5-hydroxy-1,4-naphthoquinone.

12. The use claimed in claim 10, characterized in that root bark of dried JUGLANS REGIA L. or JUGLANS NIGRA L. in finely ground form is used as the root bark of JUGLANDACEAE species.

13. The use claimed in claim 10 or 12 to 16, characterized in that root bark which has been treated with a hydrophobicizing agent is used.

14. The use claimed in claim 10, characterized in that 0.001 to 0.5% by weight 5-hydroxy-1,4-naphthoquinone, based on the water content of the cosmetic preparation, is used.

15. The use claimed in any of claims 10 to 13, characterized in that the root bark or the extract is used in such quantities that the content of 5-hydroxy-1,4-naphthoquinone is from 0.001 to 0.5% by weight, based on the water content of the cosmetic preparation.

16. The use claimed in any of claims 10 to 15, characterized in that the 5-hydroxy-1,4-naphthoquinone is used in a dental or oral hygiene preparation, a deodorant or a foot powder.

17. The use claimed in claim 16, characterized in that the dental or oral hygiene preparation is a tooth paste, a dental chewing gum, a tooth powder or a mouthwash.

## Revendications

1. Préparation cosmétique ayant des propriétés germicides, caractérisée en ce qu'elle contient de la 5-hydroxy-1,4-naphtoquinone sous forme de parties végétales qui présentent une teneur en 5-hydroxy-1,4-naphtoquinone et dont la teneur en eau a été abaissée à une valeur de 0,3 à 0,5 % maximum, les parties végétales étant des écorces de racines de genres des juglandacées.

2. Préparation cosmétique ayant des propriétés germicides; caractérisée en ce qu'elle contient de la 5-hydroxy-1,4-naphtoquinone sous forme d'un extrait, l'extrait ayant été obtenu par extraction d'écorces de racines de genres des juglandacées qui présentent une teneur en 5-hydroxy-1,4-naphtoquinone.

3. Préparation cosmétique selon la revendication 1, caractérisée en ce que les écorces de racines de genres de juglandacées JUGLANS REGIA L. ou JUGLANS NIGRA L. séchés sont sous forme finement broyée.

4. Préparation cosmétique selon la revendication 1 ou 3, caractérisée en ce qu'elle contient des écorces de racines qui ont été traitées avec une substance conférant un caractère hydrophobe.

5. Préparation cosmétique selon la revendication 1, caractérisée en ce qu'elle contient 0,001 à 0,5 % en poids de 5-hydroxy-1,4-naphtoquinone par rapport à la teneur en eau de la préparation cosmétique.

6. Préparation cosmétique selon l'une des revendications 1 à 4, caractérisée en ce qu'elle contient les écorces de racines ou l'extrait en une quantité telle que la teneur en 5-hydroxy-1,4-naphtoquinone est de 0,001 à 0,5 % en poids par rapport à la teneur en eau de la préparation cosmétique.

7. Préparation cosmétique selon l'une des revendications 1 à 6, caractérisée en ce qu'elle est sous forme d'un produit d'hygiène dentaire et buccale, d'un désodorisant ou d'une poudre pour les pieds.

8. Préparation cosmétique selon la revendication 7, caractérisée en ce que le produit d'hygiène dentaire et buccale est une pâte dentifrice, une gomme à mâcher pour l'hygiène dentaire, une poudre dentifrice ou une eau dentifrice.

9. Procédé d'obtention d'une préparation cosmétique ayant des propriétés germicides selon au moins une des revendications 1 à 8, caractérisé en ce qu'on mélange à la formulation de base connue de la préparation cosmétique des écorces de racines de genres des juglandacées qui présentent une teneur en 5-hydroxy-1,4-naphtoquinone ou un extrait obtenu à partir de celles-ci.

10. Utilisation de la 5-hydroxy-1,4-naphtoquinone sous forme de parties végétales qui présentent une teneur en 5-hydroxy-1,4-naphtoquinone et dont la teneur en eau a été abaissée à une valeur de 0,3 à 0,5 % maximum, comme germicide pour des préparations cosmétiques, les parties végétales étant des écorces de racines de genres des juglandacées.

11. Utilisation de la 5-hydroxy-1,4-naphtoquinone sous forme d'un extrait, l'extrait ayant été obtenu par extraction d'écorces de racines de genres des juglandacées qui présentent une teneur en 5-hydroxy-1,4-naphtoquinone.

12. Utilisation selon la revendication 10, caractérisée en ce que l'on utilise comme écorces de racines de genres des juglandacées des écorces de racines de JUGLANS REGIA L. ou de JUGLANS NIGRA L. séché sous forme finement broyée.

13. Utilisation selon la revendication 10 ou les revendications 12 à 16, caractérisée en ce que l'on utilise des écorces de racines qui ont été traitées avec une substance conférant un caractère hydrophobe.

14. Utilisation selon la revendication 10, caractérisée en ce que l'on utilise 0,001 à 0,5 % en poids de 5-hydroxy-1,4-naphtoquinone, par rapport à la teneur en eau de la préparation cosmétique.

15. Utilisation selon l'une des revendications 10 à 13, caractérisée en ce que l'on utilise les écorces de racines ou l'extrait en une quantité telle que la teneur en 5-hydroxy-1,4-naphtoquinone est de 0,001 à 0,5 % en poids par rapport à la teneur en eau de la préparation cosmétique.

16. Utilisation selon l'une des revendications 10 à 15, caractérisée en ce que l'on utilise la 5-hydroxy-1,4-naphtoquinone dans un produit d'hygiène dentaire et buccale, un désodorisant ou une poudre pour les pieds.

17. Utilisation selon la revendication 16, caractérisée en ce que le produit d'hygiène dentaire et buccale est une pâte dentifrice, une gomme à mâcher pour l'hygiène dentaire, une poudre dentifrice ou une eau dentifrice.